# EUROPEAN PATENT SPECIFICATION

(11) **EP 0 343 311 B1**
(45) Date of publication and mention of the grant of the patent: **06.10.1993**
(21) Application number: 88830235.3
(22) Date of filing: 23.05.1988
(51) Int. Cl.: A61B 17/32, A61B 19/02

(54) **Blade removal and/or mounting mechanism and dispenser, extractor-disposal apparatus including same**
Klingenabnahme- und/oder -befestigungsmechanismus sowie diesen Mechanismus enthaltende Spender- und Extraktions-/Wegwerfvorrichtung
Mécanisme pour ôter et/ou monter les lames et dispositif distributeur et d'extraction, et mise au rebut comprenant ce mécanisme

(43) Date of publication of application: 29.11.1989
(73) Proprietor: THE RESEARCH FOUNDATION OF STATE UNIVERSITY OF NEW YORK, Albany, New York 12201-0009 (US)
(72) Inventor: Pollak, B.Stanley, Huntington,New York 11768 (US); Blasnik, William, Englewood, New Jersey 07631 (US)
(74) Representative: Pederzini, Paolo

(56) References cited:
- EP-A- 0 005 052
- EP-A- 0 034 949
- EP-A- 0 242 035
- WO-A-83/00454
- GB-A- 2 035 186
- US-A- 4 106 620
- US-A- 4 180 162
- US-A- 4 378 624
- US-A- 4 730 376
- US-A- 4 746 016

## Description

The present invention relates to the art of cutting blade dispensing and disposal, and, in particular, to a mechanism for blade removal and/or mounting used to provide convenient storage, dispensing, and disposal apparatus for cutting blades which can be mounted on blade handles by means of a mating elongated slot and boss.

It is known in the art of cutting to provide disposable blades, such as surgical scalpel blades, commercially in several sizes, in sterile or non-sterile packaging, which are adapted to fit conventional metal scalpel handles of various sizes to form knives used for a variety of purposes in hospitals, in research laboratories, and in science departments in schools and universities. Relative to the use in hospitals, such blades are used in surgery, pathology laboratories, etc. Typically, commercially available surgical blades have a sharpened tip and cutting edge portion and a shank portion extending rearwardly therefrom. The shank portion of the blade is provided with an elongated aperture which is shaped and adapted to receive a mating elongated boss formed on the forward or attaching tip of a scalpel handle.

Generally, the elongated handle-engaging aperture of the blade can have a widened rear portion and a narrowed forward portion, the widened rear portion initially receiving the engaging boss of the scalpel handle guiding the boss forward into the narrowed forward portion of the aperture. The boss is undercut such that the edges of the narrowed forward portion of the aperture are engaged between a scalpel handle and the undercut surface of the boss. When the boss is completely inserted within the blade aperture, the rear edge of the blade aperture can be snapped over the rear of the engaging boss, thus achieving locking engagement between the blade and the scalpel handle.

In order to remove a blade of this nature from a scalpel handle, the rear edge of the blade must be separated from the handle to disengage the rear end of the blade aperture from the rear end of the boss so that the blade can then be pushed forward until the undercut boss clears the narrowed forward portion of the blade aperture permitting the blade to be cleared of the handle.

A sharp edge is essential in conducting a surgical operation. However, blades tend to lose their edge very quickly in such procedures so it is common to use several blades during a single surgical procedure. Thus, removal of a used blade from the handle and replacement of the blade with a new sterile blade is a frequent occurrence in the course of a surgical procedure.

The construction and operation of mounting and dismounting scalpel blades on blade handles present problems in that the handling of such sharp blades for mounting and removal purposes can easily cause injury to the handler. Accordingly, in the past it has been known to provide devices for removing the blade without the necessity of touching them with the hand.

For example U.S. Patent No 3,172,316 to Grieshaber shows a blade removing tool formed from tubing and having an elongated handle. One end is flattened somewhat so as to provide flat opposed surfaces in which are formed opposed channel-like grooves which function as guide tracks for the boss portion of the scalpel when the tool and the scalpel boss portion are assembled. Extending longitudinally from one flat surface of the flattened end portion of the handle are two spaced-apart prongs which are inclined upwardly a slight amount. The space between the prongs is such as to permit the slender boss portion of the scalpel handle to pass therebetween, the free end of each prong being offset upwardly. The blade end of the scalpel handle can be initially inserted longitudinally through the open flat end of the tube between the channel-like grooves. The offset ends of the tool prong slidably engaging the under surface of the blade until they engage behind the innermost edge of the blade adjacent the scalpel handle. The inclination of the prongs causes the innermost edge portion of the accommodated blade to be flexed upwardly so that each edge portion thereof will clear the slender boss portion of the scalpel when the scalpel is withdrawn longitudinally from the flattened end of the tool. The blade of the scalpel is held in place by the offset ends of the prongs during withdrawal of the scalpel handle.

U.S. Patent No. 4,180,162 to Magney shows a combination dispenser-disposal cartridge for a surgical blade which includes an elongated open-top box with means for receiving and positioning the blade in a curved position to accept the mating boss of a scalpel handle. The box also includes means for stripping a used blade from the scalpel handle and retaining it within the box for disposal. The blade is packed in the box so that it curvingly extends from the forward tip towards the shank tip to facilitate insertion of a boss of a scalpel handle into the elongated slot, which is then moved forward and withdrawn with the blade mounted on the handle. Removal of the blade from the scalpel handle is effected by inserting the scalpel handle into the aperture end of the box until the tip of the blade is engaged between the side wall and a projection extending downwardly from the top of the box at which point the handle is moved to the right so that the rear edge of the blade is stripped away from the narrowed forward portion of the scalpel handle by engagement against an upstanding blade disengaging projection formed integrally with the floor of the box. The Magney combination dispenser-disposal of the box. The Magney combination dispenser-disposal cartridge can prove to be somewhat ineffective in use, resulting in surgeons or assistants resorting to manual removal of the blade from scalpel handles.

Other devices include the scalpel blade remover shown in U.S. Patent No. 4,378,624 to Klingerberg which includes a fixed block in combination with a second movable block having a slot between such blocks and a tab provided on the movable block to engage an end of the blade and move it relative to the body of the blade to disengage it from the handle. The blocks are mounted on a supporting surface beneath which a sterile disposal box may be disposed.

U.S. Patent No. 4,318,473 to Sandel shows a surgical blade removal and disposal device which operates by inserting a handle with a blade mounted thereon through a guide means so that the rear of the blade is disposed over spaced apart shoulders after which the handle is urged downward tending to bow the blade, thus disengaging the rear of the inserted portion from the rear edge of the blade slot to allow the handle to slide relative to the blade. The blade tends to move with the handle as a result of friction between the blade and the handle unit until it encounters the front wall of a stop which prevents further movement of the blade rearwardly. One of the problems encountered with the Sandel blade removal device is the severe bend imposed on the blade when it is fit over the spaced-apart shoulders to guide the blade to the rear stop. This causes a high degree of friction between the blade slot and the handle boss making removal of the blade very difficult.

U.S. Patent No. 4,344,532 to Eldridge, Jr., et al. discloses a surgical blade remover having a wedge shaped support member which tapers from its front side to its back side. The support has one or more mutually parallel latitudinal slots open at one end and along their length extending from the front side of the support to its interior. The slots are sized to receive the tang of the blade holder while preventing the blade itself from passing therethrough and the surface of each of the support members bordering the slot is covered with an adhesive which holds the blade in place while the handle is pivoted downward in the slot away from the blade. In certain embodiments, the slots are shown to have modifications contoured to compliment the shape of the blade and/or to provide a notch to receive a portion of the hilt of the blade in order to assist in blade removal.

U.S. Patent No. 4,120,397 to Neumann shows a unit for accommodating disposable blade-like articles in which the underside of a blade such as a scalpel blade, slidably engages a resilient tongue-like element which is deflected upwardly. The tongue-like element has mounted thereon cam means having surfaces which can be pushed against the blade to unseat the rear of the blade away from a boss on the blade handle. Once the tongue-like member is fully depressed thereby deflecting the rear of the blade downwardly, the scalpel handle can be removed, abutting the rear of the blade against the inside surface of a panel, thus unseating the blade from the boss and disassembling the blade from the handle.

U.S. Patent No. 4,106,620 to Brimmer, et al, shows a surgical blade dispenser and disposal assembly which includes blades individually positioned and supported within the box between a slot in a forward wall and a slot in a rearward wall which holds the blades in such a fashion as to slightly deform them in a lateral curve for receipt of a boss of a surgical blade holder in the elongated slot formed in the body of the blade. The blades can be removed by insertion of the blade bearing handle through the aperture and wedged rearwardly against projecting ears. The handle is then moved laterally to separate the rear of the blade from the boss and remove the handle completely off the blade. This device has proved to be cumbersome and the removal apparatus does not provide for efficient removal of the blades.

It is therefore an object of the present invention to provide a scalpel blade dispenser by which scalpel blades can be quickly and easily mounted onto a scalpel blade handle.

It is another object of the invention to provide a scalpel blade extractor which facilitates easy removal of the blade from a blade handle.

Another object of the invention is to provide a scalpel blade dispenser holder which gives quick and easy access to a variety of scalpel blades.

Commensurate with the previous object of the invention, it is a further object to provide a device by which stored blades can be easily identified.

A further object of the present invention is to provide for safe storage of scalpel blades during the course of surgical operations.

Another object of the invention is to facilitate the safe disposal of used and contaminated scalpel blades.

Another object of the invention is to permit the quick and accurate inventory of scalpel blades usually conducted at the conclusion of surgical operations.

Other and further objects of the present invention will become apparent to those skilled in the art in view of the disclosure of the invention as set forth herein.

The present invention includes a mechanism which can be used for both mounting and removing a blade having an elongated slot mounting means from a blade handle which has a mounting boss for insertion into the elongated slot. The mechanism includes a handle guide which forms one side of a passageway for insertion of the blade handle, the handle guide having a flexible body portion which is sufficiently flexible to allow deflection of the handle for withdrawal of the boss out of mating relationship with the elongated slot of the blade. Another element of the mechanism is a blade extracting means fixed opposite the handle guide which forms a second side of the passageway, the extracting means having a blade retaining projection arranged adjacent the passageway which can be actuated to prevent withdrawal of a blade from the passageway. The mechanism further includes a actuation means fixed for actuation of the blade extracting means upon deflection of the handle sufficiently to disengage the boss out of a mating relationship with the elongated slot. As a consequence a blade mounted on a handle can be removed therefrom when the handle is withdrawn from the passageway while the extracting means is being actuated.

A device of the present invention can also include a well for holding a blade in a position for mounting on a handle as well as for receiving a blade once used. Alternatively, the well can be used as a disposal receptacle when the mechanism is used merely as a blade disarmer. When it is contemplated to provide a dispensing/disposal apparatus, the well can also include a blade support which holds a blade in a mounting position while preserving the blade's cutting edge.

Referring to the individual elements of the mechanism, the blade extracting means includes a flexible arm extending from a point at one end of the passageway and extends along the passageway to an operative end, the blade retaining projection being fixed at the operative end so that it is positioned out of the passageway in the unactuated condition and so that it can be drawn over the passageway when actuated. The actuation means, in turn, includes a latch means secured to, for example, the handle guide, the latch means including a yoke extending around the passageway and a yoke tab extending around the blade removal hook arm whereby the tab is drawn into actuating contact with the arm when the handle guide is deflected away from the blade extracting means. Alternatively, the latch means can be secured to the blade removal arm while the yoke extends around the passageway and the yoke tab extends around the handle guide. The yoke tab can extend around either the arm or the handle guide a distance sufficient to permit insertion of the handle through the passageway without actuating the actuating arm.

The mechanism can be included in a housing to provide a combination blade dispenser and disposal device. The housing can be a sterilizable clear container wherein the blades can be visibly packaged and sterilized. A reclosable lid can be formed over the entire set of blade openings with a weakened portion attaching it to the housing on one side and an opening on the opposite side for articulable movement over the blades. Preferably, the device can include at least four blades for use in an operating environment.

It has been found that the device can preferably be from about 5cm (2") to about 7cm (2 3/4") high, from about 7.5cm (3") to about 10cm (4") long, and from about 4.5cm (1 3/4") to about 7cm (2 3/4") wide at the base, and can be provided with an adhesive on the bottom surface for securing the device in place during use.

As a result of the present invention a medical operating team can be provided with quick and easy access to a variety of scalpel blades in the device which permit the safe handling of the blades by mechanically mounting and dismounting as required. The blades can be identified as new or used and the device permits a blade count at the conclusion of the operation to be performed more quickly and accurately while allowing for the safe storage and disposal of the used scalpel blades.

In another embodiment, the invention can be used to provide a blade disposal container which contains the mechanism described above for disarming a blade from a handle.

For a better understanding of the present invention, together with other and further objects, reference is made to the following description, taken in conjunction with the accompanying drawings, and its scope will be pointed out in the appended claims.

Preferred embodiments of the invention have been chosen for purposes of illustration and description and are shown in the accompanying drawings wherein:
- Figure 1 is a perspective view of a blade dispensing/disposal apparatus in accordance with the present invention:
- Figure 2 is a perspective view of a handle with a blade mounted thereon;
- Figure 3 shows the relationship of the blade and the handle in the disassembled condition;
- Figure 4 is a perspective view of the operative mechanism of the present invention with the scalpel blade shown in phantom in order to depict the relationship of the blade to the mechanism;
- Figure 5 is a side elevational cross-section of the apparatus shown in Figure 1 taken along lines 5-5;
- Figure 6 is an exploded isometric view of a handle guide and latch hook assembly of the operative mechanism;
- Figure 7 is an exploded isometric of a blade hook assembly portion of the mechanism;
- Figure 8 shows an alternative embodiment of the operative mechanism of the present invention;
- Figure 9 is an end view of the apparatus shown in Figure 1 with partial cutaway to depict the operation of the lid and the blade usage indicator tab;
- Figured 10 and 11 show the steps involved in mounting a blade onto a blade handle;
- Figure 12 shows the operation of the mechanism for removal of the blade from the handle;
- Figure 13 is yet another embodiment of the present invention which is useful as a blade disarming and disposal apparatus; and
- Figure 14 is a cross-section of the embodiment shown in Figure 13 taken along lines 14-14.

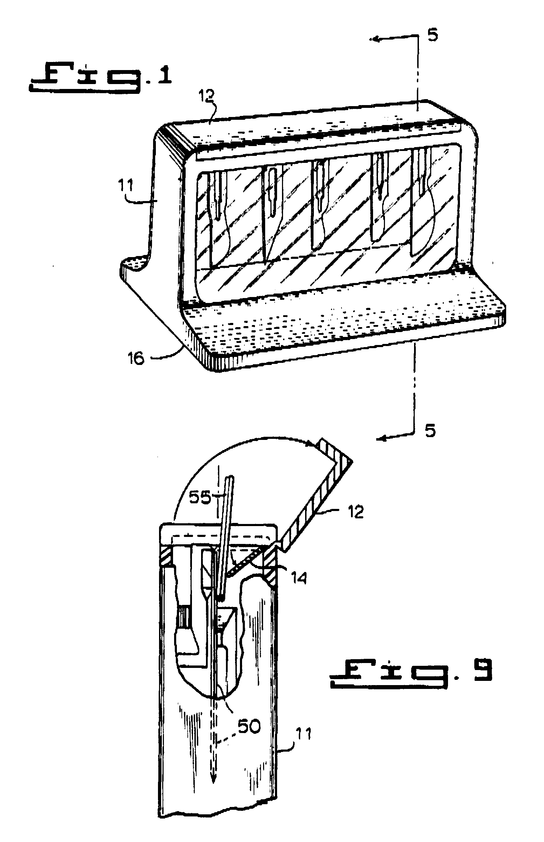

To the extent possible, the same numerals will be used throughtout the figures when referring to the same parts. Referring to Figure 1 there can be seen a device constructed in accordance with the present invention for packaging a series of blades, such as scalpel blades, for mounting on a scalpel blade handle, and for dismounting the blade from the handle and disposal after use. In Figure 1 the device is shown with five blades which can be packaged, sterilized, and delivered to the operating environment for selection and use by the operating team. The dispenser can have a transparent housing 11 whereby the different size and type of blades can be conveniently viewed by the surgeon or assistant.

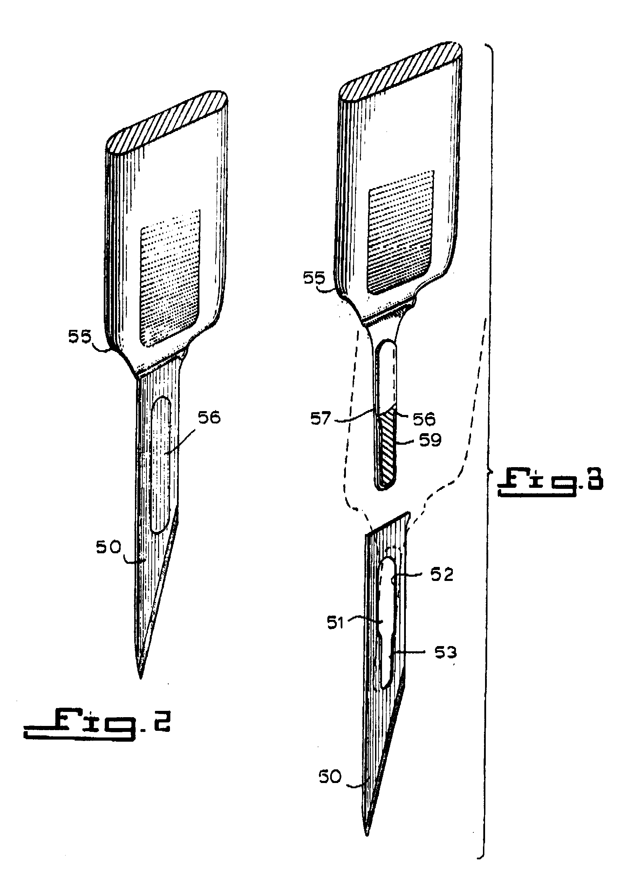

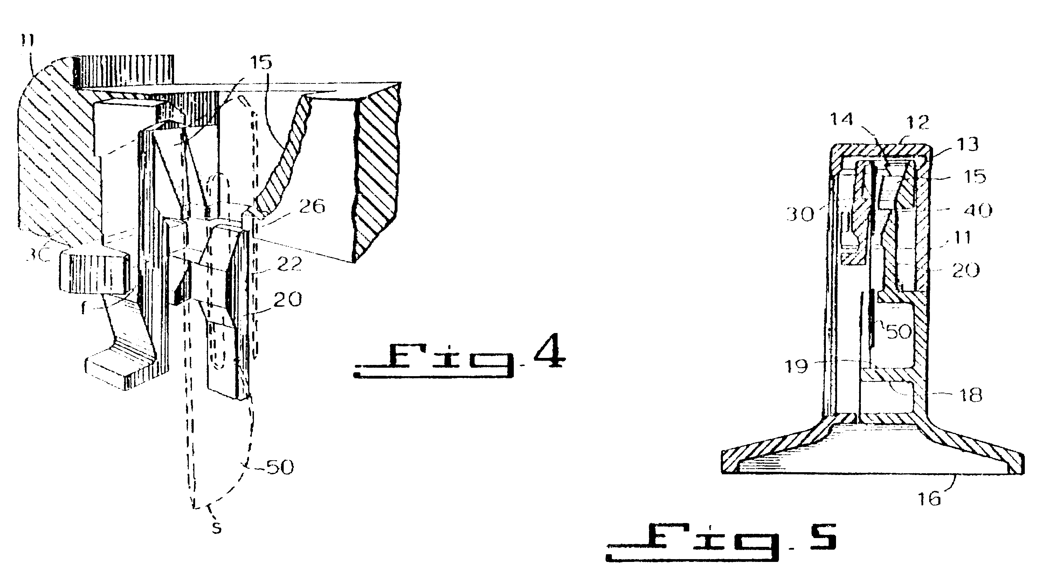

Figure 2 and 3 show the relationship of the scalpel blade to the scalpel blade handle in a fully assembled position in Figure 2, and in a disassembled configuration in Figure 3. Referring to these figures there can be seen a blade 50 having an elongated aperture 51 with a reduced sized section 53 and an enlarged section 52. It is also known that the elongated aperture can have three variations in aperture size rather than merely two as shown in Figure 3.

A blade handle 55 is shown with an elongated forward portion 57 provided with a peripheral mounting slot 59. The peripheral slot 59 can be formed by provision of a raised boss 56 having the undercut peripheral slot 59. The boss 56 extends outwardly from the forward end portion 57. Referring specifically to Figure 3 there is shown a partial cutaway of the boss 56 to show the peripheral mounting slot 59 formed as an undercut of the boss 56. In order to mount the blade 50 onto the handle 55, the raised boss portion 56 must be directed into the enlarged portion 52 of the elongated aperture 51 and thrust forward so that the entire body of raised boss 56 is inserted into the elongated aperture 51. The narrowed portion 53 of the elongated aperture is frictionally engaged by the peripheral slot 59 to secure the blade 50 on the handle 55. It is noted that the blade is secured on the handle when the rear portion 52 of aperture 51 drops over the rear portion of the raised boss 56 and snaps into mating relationship therewith.

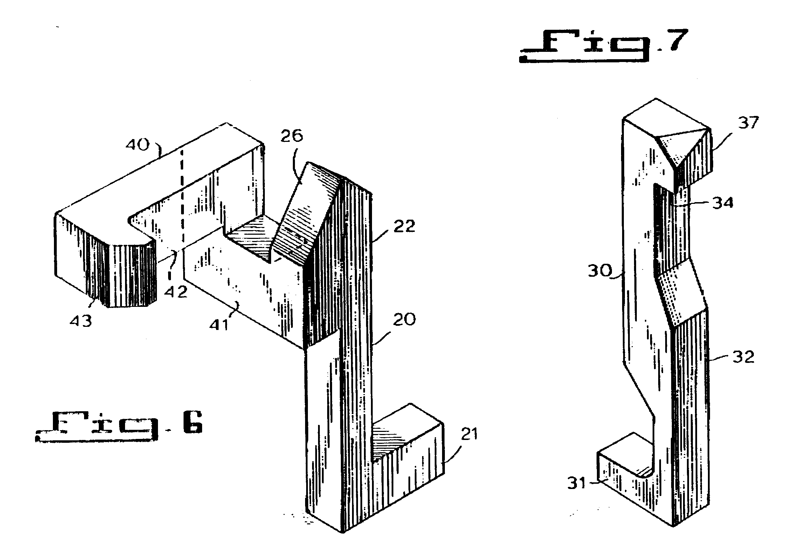

Referring to Figure 4, the operative mechanism of the present invention is show with a blade 50 shown in phantom therein. Reference is also made to Figure 6 and 7 to show the different elements of the operative portion shown in exploded isometric view. In particular, the operative mechanism includes a handle guide assembly 20 having a lower frame 21, which can be part of a molded base portion, and an upper handle guide 22 having a guiding surface 26. The handle guide assembly is fixed within housing 11 opposite a blade hook assembly 30 to form one side of a passageway for the blade and blade handle.

The blade hook assembly 30, in turn, includes a housing support part 31, as well as a leg portion 32 which extends upwardly to a blade removal hook 37 having a blade removal surface 34.

A third portion of the operative mechanism of the present invention is a latch hook assembly 40 which is rigidly fixed to the handle guide 22 by means of a latch bar 41 and continues perimetrically around the blade passage by means of a latch extension 42. At the end of the latch extension 42 is a latch hook 43, which extends around the leg 32 a distance "f" from the leg 32 to permit deflection of the handle guide assembly 20 a distance sufficient to permit insertion of a handle tip in the passageway for mounting a blade thereon.

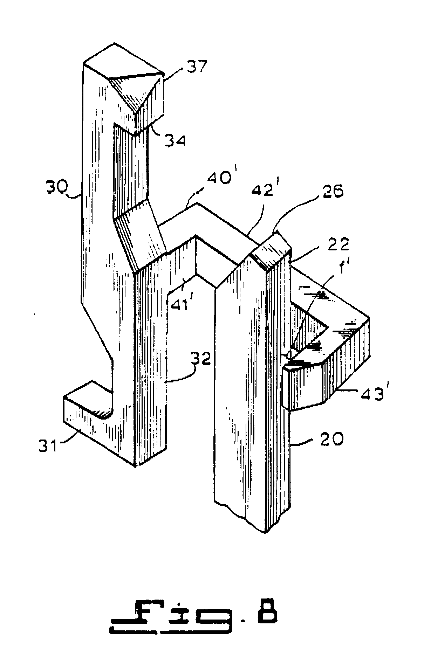

In Figure 8, an alternative embodiment of the present invention is shown with the latch hook assembly rigidly affixed to the blade removal hook rather than the handle guide assembly. These parts of the configuration shown in Figure 8 which have been rearranged are designated with primed numbers to indicate that they parts with similar function, but with a different configuration.

Referring to Figure 5 there can be seen a side elevational view in cross-section of the apparatus as shown in Figure 1 taken along lines 5-5 which shows the relationship of the operative portions of the mechanism to the housing 11. In this view the blade is shown resting on a blade platform 18, which can include a blade support projection 19. The support projection 19 is located on the platform 18 at a position wherein the blade rests thereon at location "s" ( seen in Figure 4) which is essentially unused during a procedure, e.g. usually between the point and the cutting "belly" of the blade.

Further features of the present invention include in the preferred mode, a lid 12 preferably, having a living hinge 13, which can be raised rearwardly (see Figure 9) to expose a blade use indicator tab 14 located immediately above the packaged blade. In operation, when the handle is inserted to mount the blade thereon, the blade indicator tab 14 is depressed downwardly to be captured between converging shoulders 15 provided in the housing, so that once depressed, use of the blade is memoralized by the downwardly-retained tab.

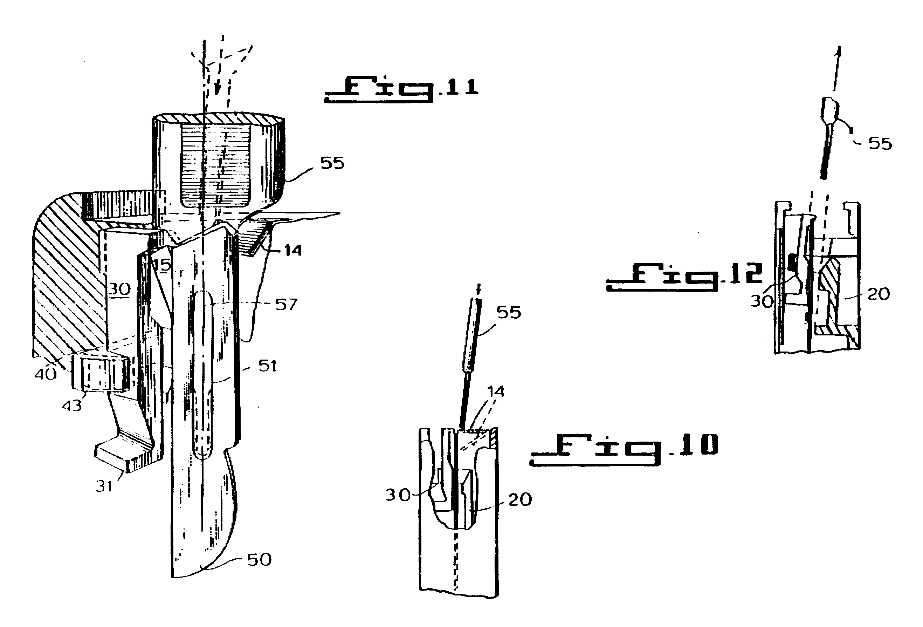

The operation by which a blade is mounted on the handle is shown in Figures 10 add 11. In particular, in Figure 10, the front portion 57 of a blade handle 55 is thrust downwardly to depress the blade indicator tab 14 and to gain access into the passageway between the handle guide assembly 20 and the hook removal assembly 30. Referring now to Figure 11, the handle can be seen as it has been engaged with the elongated aperture 51 of the blade 50 The tab 14 which has been depressed is retained in the depressed condition by frictional fit between converging shoulders 15. Since there is a distance "f" between the leg 32 and the latch hook 43, the handle guide can be deflected to a certain extent without activating the blade removal assembly 30. Once the blade has been secured on the front end 57 of the handle 55, the entire assembly can be vertically removed from the mechanism.

The removal of a blade from a handle is explained with reference to Figure 12. The blade and handle assembly can be inserted once again through the opening provided by the permanently depressed tab 14 and into the passageway between the handle guide assembly 20 and the blade hook assembly 30. Once the blade is fully inserted into the passageway, the handle 55 can be deflected toward the handle guide assembly 20 and away from the blade removal assembly 30, i.e., to the right as shown in Figure 12. As a result of this deflection, the handle 55 strikes against the upper handle guide 22 thereby urging the handle guide assembly with the latch hook assembly 40 rigidly fixed thereto away from the passageway. The latch hook 43, thereby traverses through the distance "f" and strikes the rear of leg 32 of blade removal hook assembly 30 thereby urging it over the passageway as shown in Figure 12. When the blade handle is deflected out of the passageway, the rear portion of the raised boss 56 is simultaneously pulled from the rear portion of the elongated aperture 51 thus, permitting a disengaging movement of the handle from the blade.

In the present mechanism the disengaging movement is a vertically upward pull of the handle out of the passageway, which, in the condition shown in Figure 12 causes the top end of the blade 50 to strike against the blade removal surface 34 thereby preventing the blade from being lifted from the passageway. It should be noted that since only a slight deflection of the handle away from the blade is required to disengage the boss 56 from the aperture 51, and to simultaneously actuate the blade removal assembly, there is but a minimal increase in the friction between the narrow portion 53 of the blade aperture 51 and the undercut peripheral slot 59 of the boss 56. Thus, as a result of the actuation of the removal assembly 30 and the low-fractional-fit condition of the blade 50 on the mounting boss 56, the handle can be very easily withdrawn and disassembled from the blade without touching the blade itself.

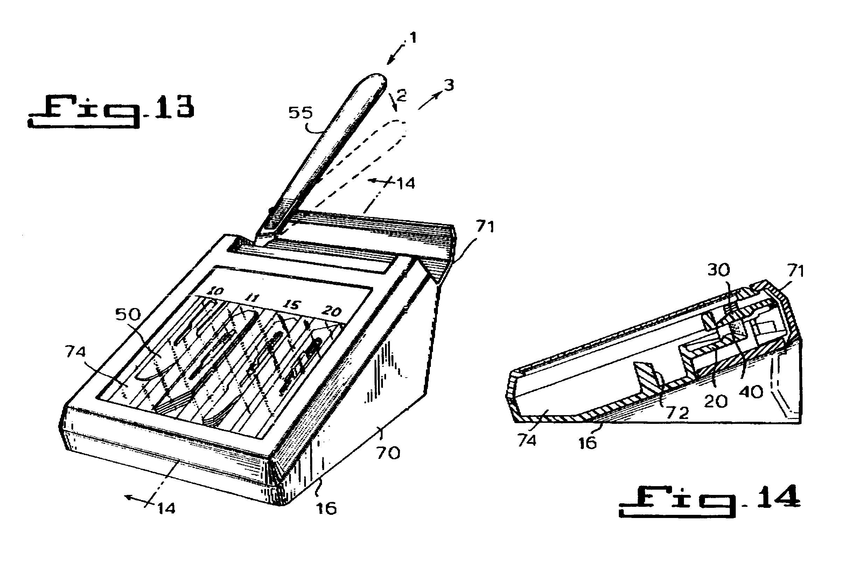

Referring to Figure 13 there is shown a further embodiment of the present invention which can be used merely as a disposal apparatus for removal and storage of used blades. In particular there is shown device 70 with a lid 71 which can be rotated rearwardly from the top of the apparatus 70 as shown in phantom in Figure 14. Once the lid is opened, a fully assembled blade and handle can be inserted into the mechanism and manipulated as shown in Figure 13 to remove a blade. Additionally, in the configuration shown in Figures 13 and 14 a blade retaining post 72 can be provided in receiving wells 74 in order to maintain the blades therein in a coherent stack.

In either of the preferred embodiments, when the housing is made of a transparent material, the blades disposed of can be easily inventoried after a procedure to facilitate accountability. Further with regard to the preferred embodiments, the composite housing unit can have an adhesive bottom 16 which holds it firmly in place for both the blade mounting and dismounting procedure as described above. The dispensing device with new blades packaged therein can be optionally covered with a sterile wrap material and subjected to sterilization procedure by use of ethylene oxide, radiation, etc.

## Claims

1. A mechanism for mounting and removing a blade (50) having an elongated slot mounting means (51) from a blade handle (55) which has a mounting boss (56) for insertion into said elongated slot mounting means (51) comprising:
- a blade housing (11) for holding said blade in a position for mounting on said blade handle (55) and for receipt of said blade upon dismounting from said handle (55),
- a handle guide (20) forming one side of a passageway into said blade housing (11) for insertion of said handle (50), said handle guide (20) having a flexible body portion being sufficiently flexible to allow deflection of said handle (55) away from said blade (50) for insertion of said boss (56) into mating relationship with said elongated slot mounting means (51) and for withdrawal of said boss (56) out of mating relationship with said elongated slot,
- blade extracting means (30) fixed opposite said handle guide (20) which forms a second side of said passageway opposite said handle guide (20), said extracting means (30) having a blade retaining projection (37) arranged adjacent said passageway which can be actuated to prevent withdrawal of a blade from said blade housing housing (11), characterized in that if further comprises
- actuation means (40) fixed for actuation of said blade extracting means (30) upon deflection of said handle (50) sufficient to disengage said boss (56) out of mating relationship with said elongated slot mounting means (51), whereby a blade (50) mounted on said handle (55) is engaged by said retaining projection (37) for removal as said handle is withdrawn from said passageway.

2. The mechanism of claim 1 wherein said blade extracting means (30) further comprises a flexible arm (32) extending from a point adjacent said blade housing (11) along said passageway to an operative end, said blade retaining projection (37) fixed at said operative end so that it is positioned out of said passageway in the unactuated condition and so that it can be drawn over said passageway when actuated whereby said blade (50) is prevented from being removed from said passageway.

3. The mechanism of claim 2 wherein said actuation means (40) comprises latch means secured to one of said handle guide (20) and said blade extracting means (30), said latch means including a yoke extending around said passageway and a latch hook (43) extending around one of said blade extracting means (30) and said handle guide (20) whereby said latch hook (43) is drawn into actuating contact with one of said extracting means (30) and said handle guide (20) when said handle guide is deflected away from said blade extracting means (30).

4. The mechanism of claim 3 wherein said latch hook (43) extends around one of said extracting means (30) and said handle guide (20) a distance sufficient to permit insertion of said handle (55) through said passageway without actuating said extracting means (30).

5. The mechanism of claim 1 wherein said blade housing (11) comprises a blade support (18) which bears a blade (50) in an upright position for mounting at a point along said blade which preserves a useable portion of said blade's cutting edge.

6. The mechanism of claim 1 which is mounted in a blade housing (11) to provide a combination blade dispenser and disposal device.

7. The mechanism of claim 5 wherein said blade housing (11) is transparent and wherein there are more than one said mechanism.

8. The mechanism of claim 7 wherein the device is wrapped and sterilized.

9. A mechanism for removing a blade (50) having an elongated slot mounting means (51) from a blade handle (55) which has a mounting boss (56) for insertion into said elongated slot mounting means (51), comprising
- a handle guide (20) forming one side of a passageway for insertion of said handle (55), said handle guide having a flexible body portion being sufficiently flexible to allow deflection of said handle (55) away from said blade (50) for withdrawal of said mounting boss (56) out of mating relationship with said elongated slot mounting means (51),
- blade extracting means (30) fixed opposite said handle guide (20) which forms a second side of said passageway opposite said handle guide (20), said extracting means (30) having a blade retaining projection (37) arranged adjacent said passageway which can be actuated to prevent withdrawal said blade (50) from said passageway, characterized in that it further comprises
- actuation means (40) fixed for actuation of said blade extracting means (30) upon deflection of said handle (55) sufficient to disengage said mounting boss (56) out of mating relationship with said elongated slot mounting means (51), whereby a blade (50) mounted on said blade handle (55) is engaged by said retaining projection (37) for removal as said handle (55) is withdrawn from said passageway.

10. The mechanism of claim 9 wherein said blade extracting means (30) further comprises a flexible arm (32) extending from a point adjacent to a blade housing (11) along said passageway to an operative end, said blade retaining projection (37) being fixed at said operative end so that it is positioned out of said passageway in the unactuated condition and so that it can be drawn over said passageway when actuated whereby said blade (50) is prevented from being removed from said passageway.

11. The mechanism of claim 10 wherein said actuation means comprises latch means (40) secured to one of said handle guide (20) and said blade extracting means, said latch means including a yoke extending around said passageway and a latch hook (43) extending around one of said blade extracting means (30) and said handle guide (20) whereby said latch hook (43) is drawn into actuating contact with one of said blade extracting means (30) and said handle guide (20) when said handle guide is deflected away from said blade extracting means (30).

12. The mechanism of claim 11 wherein said latch hook (43) extends around one of said extracting means (30) and said handle guide (20) a distance sufficient to permit insertion of said handle through said passageway without actuating said extracting means (30).

13. The mechanism of claim 9 which is mounted in a housing (11) to provide a combination blade dispenser and disposal device.

14. The mechanism of claim 12 wherein said housing (11) is transparent and wherein there are more than one said mechanism.

## Patentansprüche

1. Mechanismus Zur Befestigung und Abnahme einer Klinge (50) mit einem Langschlitzmittel (51) zur Befestigung aus einem Klingenheft (55), das einen Befestigungsvorsprung (56) zum Einschalten ins genannte Langschlitzbefestigungsmittel (51) hat, umfassend:
- ein Klingengehäuse (11) zum Halten der genannten Klinge in einer Stellung zur Befestigung am genannten Klingenheft (55) und Zur Aufnahme der genannten Klinge, als diese vom genannten Heft (55) abgezogen wird,
- eine Heftführung (20), die eine Seite eines Durchgangs innerhalb des genannten Klingengehäuses (11) zum Einschalten des genannten Heftes (50) bildet, wobei die genannte Heftführung (20) einen biegsamen Körperteil hat, der dermaßen biegsam ist, daß er die Biegung des genannten Heftes (55) in Entfernung aus der genannten Klinge (50) erlaubt, damit der genannte Vorsprung (56) in Verbindungsverhältnis mit dem genannten Langsschlitzbefestigungsmittel (51) eingeschaltet und der genannte Vorsprung (56) aus dem genannten Verbindungsverhältnis mit dem genannten Langschlitzbefestigungsmittel (51) ausgezogen wird,
- der genannten Heftführung (20) gegenüberliegend befestigte Klingenausziehungsmittel (30), die eine zweite Seite des genannten Durchgangs der genannten Heftführung (20) gegenüber bilden, wobei diese Ausziehungsmittel (30) einen die Klinge haltenden dem genannten Durchgang anliegenden Vorsprung (37) haben, der zum Hindern der Ausziehung einer Klinge aus dem genannten Klingengehäuse (11) betätigt werden kann,
dadurch gekennzeichnet, daß er ferner
- Betätigungsmittel (40) umfaßt, die für die Betätigung der genannten Klingenausziehungsmittel (30) geeignet sind, wenn das genannte Heft sich dermaßen biegt, daß es den genannten Vorsprung (56) aus dem Verbindundgsverhältnis mit dem genannten Langschlitzbefestigungsmittel (51) ausklinkt, so daß eine am genannetn Heft (55) befestigte Klinge (50) durch den genannten Haltevorsprung (37) gegriffen wird, damit sie beim Ausziehen des genannten Heftes aus dem genannten Durchgang entfernt wird.

2. Mechanismus nach Anspruch 1, wobei das genannte Klingenausziehungsmittel (30) ferner einen biegsamen Arm (32) umfaßt, der sich aus einem dem genannten Klingengehäuse (11) anliegenden Punkt längs des genannten Durchgangs bis zu einem operativen Ende erstreckt, wobei der genannte Vorsprung (37) zum Halten der Klinge am genannten operativen Ende derart befestigt ist, daß er beim Außerbetriebszustand außerhalb des genannten Durchgangs positioniert ist und daß er beim Betriebszustand über den genannten Durchgang gezogen werden kann, wodurch die Entfernung der genannten Klinge (50) aus dem genannten Durchgang verhindert wird.

3. Mechanismus nach Anspruch 2, wobei das genannte Betätigungsmittel (40) Klemmmittel umfaßt, die an je einem unter der genannten Heftführung (20) und dem genannten Klingenausziehungsmittel (30) befestigt sind, wobei die genannten Klemmmittel eine sich rings um den genannten Durchgang erstreckende Gabel sowie einen sich rings um eines unter dem genannten Klingenausziehungsmittel (30) und der genannten Heftführung (20) erstreckenden Klemmhaken (43) umfassen, wodurch der genannte Klemmhaken (43) in Betätigungsberührung mit einem unter dem genannten Ausziehungsmittel (30) und der genannten Heftführung (20) gezogen wird, als die genannte Heftführung in Entfernung aus dein genannten Klingenausziehungsmittel (30) gebogen wird.

4. Mechanismus nach Anspruch 3, wobei der genannte Klemmhaken (43) sich rings um eines unter dem genannten Ausziehungsmittel (30) und der genannten Heftführung (20) zu einem hinreichenden Abstand erstreckt, um das Einschalten des genannten Heftes (55) durch den genannten Durchgang ohne Betätigung des genannten Ausziehungsmittels (30) zu erlauben.

5. Mechanismus nach Anspruch 1, wobei das genannte Klingengehäuse (11) einen Klingenhalter (18) umfaßt, der eine Klinge in einer vertikalen Stellung zur Befestigung an einem Punkt längs der genannten Klinge trägt, und der einen brauchbaren Teil der Schneidkante der genannten Klinge schützt.

6. Mechanismus nach Anspruch 1, der in einem Klingengehäuse (11) gelagert ist, um eine Kombination Klingenspender/-wegwerfvorrichtung zu schaffen.

7. Mechanismus nach Anspruch 5, wobei das genannte Klingengehäuse (11) durchsichtig ist und wobei es mehr als einen genannten Mechanismus gibt.

8. Mechanismus nach Anspruch 7, wobei die Vorrichtung umhüllt und sterilisiert ist.

9. Mechanismus zum Entfernen einer Klinge (50) mit einem Langschlitzmittel (51) zur Befestigung aus einem Klingenheft (55), das einen Befestigungsvorsprung (56) zum Einschalten ins genannte Langschlitzbefestigungsmittel (51) hat, umfassend;
- eine Heftführung (20), die eine Seite eines Durchgangs zum Einschalten des genannten Heftes (50) bildet, wobei die genannte Heftführung (20) einen biegsamen Körperteil hat, der dermaßen biegsam ist, daß er die Biegung des genannten Heftes (55) in Entfernung aus der genannten Klinge (50) erlaubt, damit der genannte Vorsprung (56) aus dem Verbindungsverhältnis mit dem genannten Langschlitzbefestigungsmittel (51) ausgezogen wird,
- der genannten Heftführung (20) gegenüberliegend befestigte Klingenausziehungsmittel (30), die eine zweite Seite des genannten Durchgangs der genannten Heftführung (20) gegenüber bilden, wobei diese Ausziehungsmittel (30) einen die Klinge haltenden dem genannten Durchgang anliegenden Vorsprung (37) haben, der zum Hindern der Ausziehung der genannten Klinge (50) aus dem genannten Durchgang betätigt werden kann, dadurch gekennzeichnet, daß er ferner
- Betätigungsmittel (40) umfaßt, die für die Betätigung der genannten Klingenausziehungsmittel (30) geeignet sind, wenn das genannte Heft (55) sich dermaßen biegt, daß es den genannten Befestigunsvorsprung (56) aus dem Verbindundgsverhältnis mit dem genannten Langschlitzbefestigungsmittel (51) ausklinkt, so daß eine am genannetn Klingenheft (55) befestigte Klinge (50) durch den genannten Haltevorsprung (37) gegriffen wird, damit sie beim Ausziehen des genannten Heftes (55) aus dem genannten Durchgang entfernt wird.

10. Mechanismus nach Anspruch 9, wobei das genannte Klingenausziehungsmittel (30) ferner einen biegsamen Arm (32) umfaßt, der sich aus einem dem genannten Klingengehäuse (11) anliegenden Punkt längs des genannten Durchgangs bis zu einem operativen Ende erstreckt, wobei der genannte Vorsprung (37) zum Halten der Klinge am genannten operativen Ende derart befestigt ist, daß er beim Außerbetriebszustand außerhalb des genannten Durchgangs positioniert ist und daß er beim Betriebszustand über den genannten Durchgang gezogen werden kann, wodurch die Entfernung der genannten Klinge (50) aus dem genannten Durchgang verhindert wird.

11. Mechanismus nach Anspruch 10, wobei das genannte Betätigungsmittel Klemmmittel (40) umfaßt, die an je einem unter der genannten Heftführung (20) und dem genannten Klingenausziehungsmittel befestigt sind, wobei die genannten Klemmmittel eine sich rings um den genannten Durchgang erstreckende Gabel sowie einen sich rings um eines unter dem genannten Klingenausziehungsmittel (30) und der genannten Heftführung (20) erstreckenden Klemmhaken (43) umfassen, wodurch der genannte Klemmhaken (43) in Betätigungsberührung mit einem unter dem genannten Ausziehungsmittel (30) und der genannten Heftführung (20) gezogen wird, als die genannte Heftführung in Entfernung aus dem genannten Klingenausziehungsmittel (30) gebogen wird.

12. Mechanismus nach Anspruch 11, wobei der genannte Klemmhaken (43) sich rings um eines unter dem genannten Ausziehungsmittel (30) und der genannten Heftführung (20) zu einem hinreichenden Abstand erstreckt, um das Einschalten des genannten Heftes durch den genannten Durchgang ohne Betätigung des genannten Ausziehungsmittels (30) zu erlauben.

13. Mechanismus nach Anspruch 9, der in einem Gehäuse (11) gelagert ist, um eine Kombination Klingenspender/-wegwerfvorrichtung zu schaffen.

14. Mechanismus nach Anspruch 12, wobei das genannte Gehäuse (11) durchsichtig ist und wobei es mehr als einen genannten Mechanismus gibt.

## Revendications

1. Mécanisme pour ôter et/ou monter une lame (50) ayant des moyens de montage (51) en forme de fente allongée, dans un manche de lame (55) qui a une protubérance de montage (56) pour l'introduction dans lesdits moyens de montage en forme de fente allongée (51), comprenant:
- un logement de lame (11) destiné à contenir ladite lame en position pour le montage sur ledit manche de lame (55) et à recevoir ladite lame quand elle a été ôtée dudit manche (55),
- un élément de guidage (20) de manche formant un côté d'un passage dans ledit logement de lame (11) pour l'introduction dudit manche (50), ledit élément de guidage (20) de manche ayant une partie de corps flexibile, de flexibilité suffisante à permettre le fléchissement dudit manche (55) à l'écart de ladite lame (50) pour l'introduction de ladite protubérance (56) de manière à épouser ladite fente allongée (51) et l'enlèvement de la dite protubérance hors de l'engagement avec ladite fente allongée,
- des moyens d'extraction de lame (30) fixés à l'opposé dudit élément de guidage (20) de manche lesquels forment un second côté dudit passage en position opposée par rapport audit élément de guidage de manche (20), lesdits moyens d'extraction (30) ayant une saillie (37) de retenue de la lame disposée adjacente audit passage laquelle peut être actionnée pour empêcher l'enlèvement de la lame dudit logement de lame (11),
caractérisé en ce qu'il comporte en outre;
- des moyens d'actionnement (40) fixés pour l'actionnement desdits moyens d'extraction (30) de lame après le fléchissement dudit manche (50) d'une quantité suffisante à dégager ladite protubérance (56) hors de correspondance avec lesdits moyens de montage en forme de fente allongée (51), de sorte qu'une lame (50) montée sur ledit manche (55) est engagée par ladite saillie de retenue (37) pour l'enlèvement quand ledit manche est extrait dudit passage.

2. Mécanisme selon la revendication 1, caractérisé en ce que lesdits moyens d'extraction de lame (30) comportent en outre un bras flexible (32) s'étendant depuis un point adjacent audit logement de lame (11) le long dudit passage jusqu'à une extrémité opératoire, ladite saillie de retenue de lame (37) étant fixée à ladite extrémité opératoire de sorte qu'elle est positionnée au-dehors dudit passage dans la condition non actionnée et qu'elle peut être tirée sur ledit passage quand actionnée, ladite lame (50) ne pouvant ainsi être enlevée dudit passage.

3. Mécanisme selon la revendication 2, caractérisé en ce que lesdits moyens d'actionnement (40) comportent des moyens de verrouillage fixés à l'un desdits élément de guidage de manche (20) et moyens d'extraction de lame (30), lesdits moyens de verrouillage comprenant un joug s'étendant autour dudit passage et un crochet de verrouillage (43) s'étendant autour de l'un desdits moyens d'extraction de lame (30) et élément de guidage de manche (20), de sorte que ledit crochet de verrouillage (43) est tiré en contact d'actionnement avec l'un desdits moyens d'extraction (30) et élément de guidage de manche (20), quand ledit élément de guidage de manche est pliée à l'écart desdits moyens d'extraction de lame (30).

4. Mécanisme selon la revendication 3, caractérisé en ce que ledit crochet de verrouillage (43) s'étend autour de l'un desdits moyens d'extraction (30) et élément de guidage de manche (20) sur une distance suffisante à permettre l'introduction dudit manche (55) à travers ledit passage sans actionner lesdits moyens d'extraction (30).

5. Mécanisme selon la revendication 1, caractérisé en ce que ledit logement de lame (11) comporte un support de lame (18) portant une lame (50) en une position verticale pour le montage à un point le long de ladite lame lequel protège une partie utilisable de ladite lame.

6. Mécanisme selon la revendication 1, lequel est monté dans un logement de lame (10) pour fournir une combinaison de distributeur de lame et dispositif de mise au rebut.

7. Mécanisme selon la revendication 5, caractérisé en ce que ledit logement de lame (11) est transparent et en ce qu'on prévoit plus d'un de ces mécanismes.

8. Mécanisme selon la revendication 7, caractérisé en ce que le dispositif est enveloppé et stérilisé.

9. Mécanisme pour ôter une lame (50) ayant des moyens de montage en forme de fente allongée (51) d'une manche de lame (55) qui a une protubérance de montage (56) pour l'introduction desdits moyens de montage en forme de fente allongée (51), comprenant;
- un élément de guidage de manche (20) formant un côté d'un passage pour l'introduction dudit manche (55), ledit élément de guidage de manche ayant une partie de corps flexible, de flexibilité suffisante à permettre audit manche (55) de se fléchir a l'écart de ladite lame (50) pour l'extraction de ladite protubérance de montage (56) en position de non correspondance avec lesdits moyens de montage en forme de fente allongée (51),
- des moyens d'extraction de lame (30) fixés en position opposée audit élément de guidage de manche (20), lesquels forment un second côté dudit passage a l'opposé dudit élément de guidage de manche (20), lesdits moyens d'extraction (30) ayant une saillie de retenue de lame (37) disposée adjacente audit passage laquelle peut être actionnée pour empêcher l'extraction de ladite lame (50) dudit passage,
caractérisé en ce qu'il comporte en outre:
- des moyens d'actionnement (40) fixés pour l'actionnement desdits moyens d'extraction de lame (30) après fléchissement dudit manche (55) suffisant à dégager ladite protubérance de montage (56) de sa position de correspondance avec lesdits moyens de montage en forme de fente allongée (51), de sorte qu'une lame (50) montée sur ledit manche de lame (55) est engagée par ladite saillie de retenue (37) pour l'enlèvement quand ledit manche (55) est extrait dudit passage.

10. Mécanisme selon la revendication 9, caractérisé en ce que lesdits moyens d'extraction de lame (30) comportent en outre un bras flexible (32) s'étendant depuis un point adjacent à un logement de lame (11) le long dudit passage, jusqu'à une extrémité opératoire, ladite saillie de retenue de lame (37) étant fixée à ladite extrémité opératoire de manière qu'elle est positionnée hors dudit passage dans la condition non actionnée et peut être tirée sur ledit passage quand actionnée, ce qui empêche que ladite lame (50) soit enlevée dudit passage.

11. Mécanisme selon la revendication 10, caractérisé en ce que lesdits moyens d'actionnement comportent des moyens de verrouillage (40) fixés à l'un desdits élément de guidage de manche (20) et moyens d'extraction de lame, lesdits moyens de verrouillage comprenant un joug s'étendant autour dudit passage et un crochet de verrouillage (43) s'étendant autour de l'un desdits moyens d'extraction de lame (30) et élément de guidage de manche (20), de sorte que ledit crochet est tiré en contact d'actionnement avec l'un desdits moyens d'extraction de lame (30) et élément de guidage de manche (20), quand ledit élément de guidage de manche est fléchi à l'écart desdits moyens d'extraction de lame (30).

12. Mécanisme selon la revendication 11, caractérisé en ce que ledit crochet de verrouillage (43) s'étend autour de l'un desdits moyens d'extraction (30) et élément de guidage de manche (20) sur une distance suffisante à permettre l'introduction dudit manche à travers ledit passage sans actionner lesdits moyens d'extraction (30).

13. Mécanisme selon la revendication 9, caractérisé en ce qu'il est monté en un logement (11) pour fournir une combinaison de distributeur de lame et dispositif de mise au rebut.

14. Mécanisme selon la revendication 12, caractérise en ce que ledit logement do lame (11) est transparent et en ce qu'on prévoit plus d'un de ces mécanismes.
